# EUROPEAN PATENT SPECIFICATION

(11) **EP 1 909 804 B1**
(45) Date of publication and mention of the grant of the patent: **29.02.2012**
(21) Application number: 06769018.0
(22) Date of filing: 23.06.2006
(51) Int. Cl.: A61K 31/7008, A61K 31/715, A61P 25/00, A61P 25/16, A61P 25/28, A61P 35/00

(54) **GLUCOSAMINE AND DERIVATIVES THEREOF USEFUL AS TRANSGLUTAMINASE INHIBITORS IN THE TREATMENT OF A NERVOUS SYSTEM DISORDER**
GLUCOSAMIN UND DERIVATE DAVON ALS TRANSGLUTAMINASE-INHIBITOREN ZUR VERWENDUNG IN DER BEHANDLUNG VON EINER FUNKTIONSSTÖRUNG DES NERVENSYSTEMS
GLUCOSAMINE ET SES DERIVES UTILISES COMME INHIBITEURS DE LA TRANSGLUTAMINASE DANS LE TRAITEMENT D'UN TROUBLE DU SYSTÈME NERVEUX

(30) Priority: 03.08.2005 KR 20050070862
(43) Date of publication of application: 16.04.2008
(73) Proprietor: National Cancer Center, Goyang-si Gyeonggi-do 410-352 (KR)
(72) Inventor: KIM, Soo Youl, Seoul, 137-950 (KR)
(74) Representative: Bassil, Nicholas Charles
(86) International application number: PCT/KR2006/002438
(87) International publication number: WO 2007/026996

(56) References cited:
- EP-A1- 1 201 136
- EP-A1- 1 374 873
- EP-A1- 1 543 821
- WO-A1-95/13061
- WO-A1-98/06418
- WO-A1-02/080934
- WO-A1-2004/084912
- WO-A1-2007/145405
- WO-A2-2004/069175
- DE-A1- 10 126 396
- KR-A- 20040 065 979

## Description

### Technical Field

The present invention relates to glucosamine or a glucosamine derivative of Chemical Formula 2: 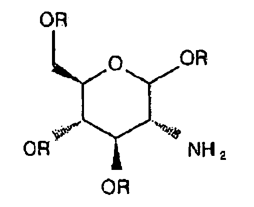 wherein, R is an acyl group having 2 to 18 carbon atoms or a straight or branched alkyl group having 1 to 5 carbon atoms, for use in the treatment of a nervous system disorder. The present invention also relates to a pharmaceutical composition comprising glucosamine or a glucosamine derivative of chemical Formula 2 for use in the treatment of a nervous system disorder.

### Background Art

Transglutaminases are protective enzymes which are responsible for blood clotting in response to tissue injury under normal conditions. However, these enzymes are also reported to play an important role in the pathological mechanism of various diseases in the absence of regulatory control in the level of expression thereof (review article. Soo-Youl Kim: New Target Against Inflammatory Diseases: Transglutaminase 2. Archivum Immunologiae & Therapiae Experimentalis 52, 332-337, 2004).

The expression of transglutaminases increases particularly upon the occurrence of various inflammatory diseases, including degenerative arthritis, diabetes, autoimmune myositis, arteriosclerosis, cerebral apoplexy, hepatocirrhosis, malignant breast cancer, meningitis, inflammatory gastric ulcer, etc.

Recently, Sohn et al. succeeded in eliciting a steroid comparable effect from a transglutaminase inhibitor consisting of a peptide in guinea pigs suffering from pollen-allergic conjunctivitis (Sohn, J., Kim, T.-I., Yoon, Y.-H., and Kim, S.-Y. Transglutaminase inhibitor: A New Anti-Inflammatory Approach in Allergic Conjunctivitis. J. Clin. Invest. 111, 121-8, 2003; Soo-Youl Kim.Transglutaminase 2 in inflammation. Front Biosci. 11, 3026-3035, 2006).

For these reasons, there is a demand for studies on transglutaminase inhibitors for use in the development of safe and effective therapeutics.

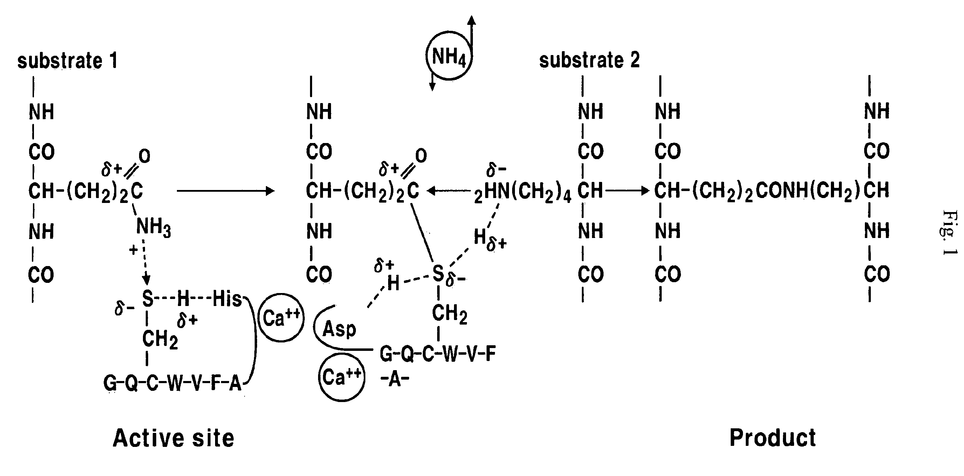

Transglutaminases (TGases, E.D.2.3.2.13) are calcium-dependent crosslinking enzymes which catalyze the linking of a glutaminyl residue of a protein/peptide substrate to a lysyl residue of a protein/peptide co-substrate. With reference to FIG. 1, the reaction mechanism catalyzed by TGase between glutamine and lysine residues is elucidated. As seen in FIG. 1, substrate 1, donating a lysine residue (acyl donor, amine receptor), and substrate 2 donating a lysine residue (acyl receptor, amine donor) form a covalent bond therebetween via an iso-peptide linkage [Nε-(γ-glutamyl)-L-lysine(GGEL)]. In FIG. 1, G-Q-C-W-V-F-A stands for the amino acid sequence of the active site of TGase. Based on a ping-pong kinetic mechanism, the enzymatic reaction may utilize an amine compound, such as monoamine, diamine, polyamine, etc., as an acyl receptor, instead of lysine residue.

When the reaction mechanism of TGase is taken into consideration, many amine compounds are expected to inhibit TGase, as can be inferred from FIG. 1. Representative of the TGase inhibitors are cystamine (Nature Genetics 18, 111-117, 1998; Nature Medicine 8, 143-149, 2002) and putrescine which have a lot of reports on in vivo and in vitro experimental results.

In addition to the above-mentioned compounds, other chemical inhibitors were developed, but are reported to be so toxic as to non-specifically inhibit other enzymes. Effective as they are in inhibiting TGase, peptide inhibitors developed prior to the present invention still have a lot of problems awaiting solutions in terms of production cost and safe practice.

International application published as WO 02/080934 relates to compositions for treating or preventing angiogenesis-dependent diseases which comprise a therapeutically effective amount of 2-amino-2-deoxy-D-glucopyranose or salt thereof.

International application published as WO 98/06418 relates to compositions comprising plant carbohydrates for dietary supplements and nutritional support for the promotion and maintenance of good health. The compositions can include phytonutrients, vitamins, minerals, herbal extracts, and other non-toxic nutrients.

International application published as WO 2004/084912 relates to a therapeutic agent for nerve damages such as those caused by spinal cord injury or nerve trauma, which comprises, as an active ingredient, a low-molecular-weight saccharide composed of at least glucuronic acid and/or N-acetylglucosamine or a pharmaceutically acceptable salt thereof.

Leading to the present invention, intensive and thorough research into TGase inhibitors, conducted by screening naturally occurring compounds which have been recognized as being safe for commercialization, resulted in the finding that glucosamine or derivatives thereof have potent inhibitory activity against TGase.

### Disclosure of the Invention

Therefore, it is an object of the present invention to provide glucosamine or a glucosamine derivative of Chemical Formula 2: 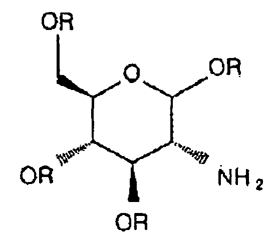 wherein, R is an acyl group having 2 to 18 carbon atoms or a straight or branched alkyl group having 1 to 5 carbon atoms, for use in the treatment of a nervous system disorder.

It is a further object of the present invention to provide a pharmaceutical composition for use in the treatment of a nervous system disorder comprising glucosamine or a derivative thereof of Chemical Formula 2 as an active ingredient.

It is an aspect of the present disclosure to provide means of inhibiting transglutaminase, featuring the use of glucosamine or a derivative thereof as an active ingredient.

It is still a further aspect of the present disclosure to provide means of treating diseases caused by the activation of transglutaminase, featuring the use of glucosamine or a derivative thereof as an active ingredient.

### Brief Description of the Drawings

The above and other objects, features and other advantages of the present invention will be more clearly understood from the following detailed description taken in conjunction with the accompanying drawings, in which:
FIG. 1 is a schematic diagram elucidating the catalytic reaction mechanism of transglutaminase.
FIG. 2 is a bar graph showing the in vitro inhibitory effect of glucosamine on transglutaminase.
FIG. 3 elucidates the inhibitory effect of glucosamine on transglutaminase in LPS-activated microglia cells by way of Western blotting with iNOS and LDH (3A) and a graph (3B) in which the % inhibition of transglutaminase is plotted against the concentration of glucosamine.
FIG. 4 elucidates the inhibitory effect of glucosamine, along with well-known transglutaminase inhibitors, on transglutaminase in SH-SY5Y neuroblastoma cells by way of Western blotting with I-κBα (4A) and a graph (4B) in which relative expression levels of I-κBα are depicted according to the kind of transglutaminase inhibitors.

### Best Mode for Carrying Out the Invention

In accordance with an aspect, the present invention pertains to a transglutaminase inhibitor based on glucosamine or a derivative thereof.

Glucosamine is a major component of chitin, a structural polysaccharide extensively found in the exoskeletons of crustaceans, such as shells of marine crabs and shrimps. Together with chitin, chitosan is a major structural constituent of the exoskeletons. Chitin is a polymer consisting of 2-acetamido-2-deoxy-β-D-glucose (N-acetylglucosamine) and chitosan is poly(β-(1,4)-glucosamine), a polysaccharide obtainable through the deacetylation of chitin. Glucosamine has the structure represented by the following Chemical Formula 1.

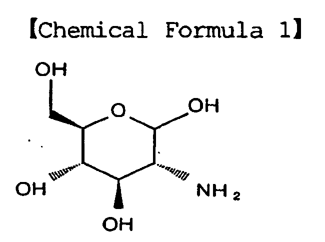

As used herein, the term "glucosamine derivative" means a glucosamine having an acyl or alkyl moiety substituted for the hydrogen of hydroxyl group, as represented by the following Chemical Formula 2. 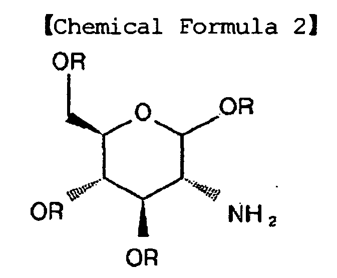 wherein, R is C2-C18 acyl, or straight or branched C1-C5 alkyl. Preferably, R is selected from an acyl group consisting of acetyl, propionyl, butyryl, pentanoyl, hexanoyl, heptanoyl, octanoyl, nonanoyl, decanoyl, undecanoyl, lauryl, tridecanoyl, myristyl, pentadecanoyl, palmitoyl, margaryl and stearyl or from an alkyl group consisting of methyl, ethyl, propyl, butyl, pentyl, isopropyl, isobutyl and sec-butyl.

As described above, the introduction of hydrophobic groups into the alcohol groups of glucosamine at positions 1, 3, 4, and 6 without change in the amine group, which is believed to be responsible for the physiological activity of glucosamine, results in glucosamine derivatives which are naturally decomposed well, non-toxic, highly adsorptive of heavy metals, and highly inhibitory of bacteria with the retention of the intrinsic physiological activity.

Unclear as it is, the inhibitory mechanism of glucosamine and derivatives thereof against transglutamases is presumably attributable to the chair conformation of glucosamine, in which the amine group binds to the active site of transglutaminase, thereby inhibiting the activity thereof.

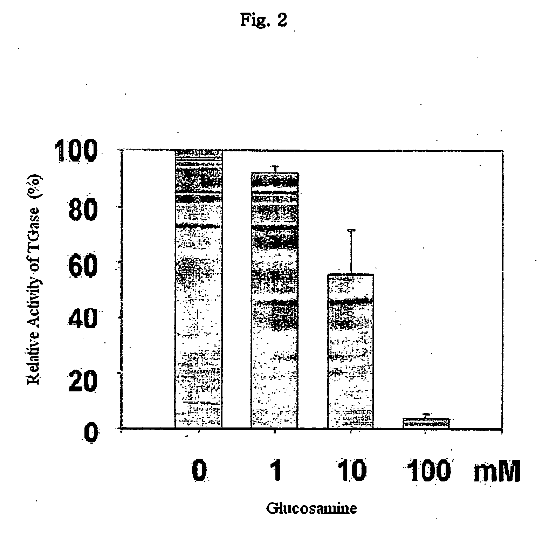

In *in vitro* experiments for transglutaminase-catalyzed reaction between ¹⁴C-labelled putrescine and succinylated casein, it was found that a higher concentration of glucosamine leads to a poorer activity of transglutaminase (FIG. 2). This result demonstrates that glucosamine, competing with putrescine, acts to inhibit the activity of transglutaminase. Consequently, glucosamine and derivatives thereof are transglutaminase inhibitors.

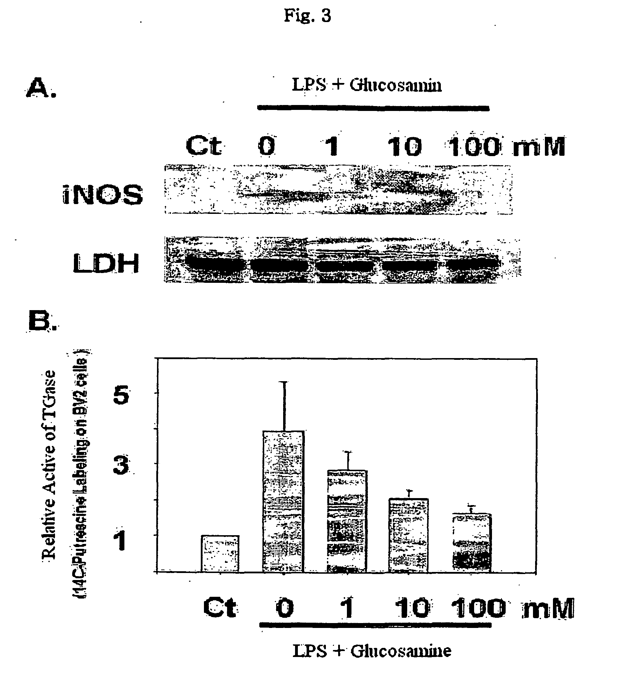

The present disclosure also provides means of inhibiting transglutaminase comprising using glucosamine or derivatives thereof. Microglia, an immune cell, increases the level of expression of transglutaminase as well as activating NF-kB therein when treated with LPS. Accordingly, co-treatment with LPS and glucosamine can reveal the relationship between the activities of transglutaminase and NF-kB (FIG. 3).

In Examples of the present invention, when the iNOS level of in vitro LPS-treated cells is increased, an inducible indicator of NF-kB, it is observed that treatment with glucosamine reduces the level of expression of iNOS (FIG. 3A). Thus, glucosamine or derivatives thereof can reduce the increased activity of transglutaminase even upon the overexpression of transglutaminase.

The present disclosure also provides a pharmaceutical composition for the prophylaxis and treatment of diseases caused by the activation of transglutaminase, comprising glucosamine or a derivative thereof, and means of treating such diseases with the pharmaceutical composition, glucosamine, or derivatives thereof.

The term "prevention" or "prophylaxis" as used herein means all of the actions in which the occurrence of any disease caused by the activation of transglutaminase is restrained or retarded by the administration of the pharmaceutical composition containing glucosamine or a derivative thereof. The term "treatment" as used herein means all of the actions in which any disease caused by the activation of transglutaminase has taken a turn for the better or been modified favorably by the administration of the pharmaceutical composition.

The diseases caused by the activation of transglutaminase include all diseases that are incurred as transglutaminase activity increases, for example, upon the overexpression of transglutaminase, and are particularly exemplified by neurological diseases and cancers.

Typical of neurological diseases are central nervous system diseases, which are associated with the death or injury of the central nervous system, such as Alzheimer's disease, multi-infarct dementia, a mixed Alzheimer/multi-infarct dementia, Parkinson's disease, hypothyroidism, Huntington's diseases, etc. These diseases are characterized by confusion, disorientation and personality disintegration with main syndromes of cognitive dysfunction, language impairment, dysfunctions in judgment, inference, temporal and spatial adaptation and learning, finally leading to the death of afflicted patients. Of them, the diseases caused by the activation of transglutaminase, e.g., the overexpression of transglutaminase in nerve tissues, are targets of the pharmaceutical composition for use according to the present invention. Particularly, the pharmaceutical composition for use in accordance with the present invention is useful in the treatment of Huntington's disease, which is associated with the overexpression of transglutaminase in the brain (Nature Medicine, Vol 8. Number 2, February 2002 pp143-149), Alzheimer's disease, which is associated with the overexpression of transglutaminase in the cerebellum and cerebral cortex (The Journal of Biological Chemistry, Vol. 274. No. 43. Issue Of October 22, pp 30715-30721), and Parkinson's disease, which is associated with transglutaminase-induced α synuclein aggregation (PNAS, February 18,2003, Vol. 100, no.4, pp2047-2052), but are not limited thereto. The present invention is applicable to the treatment of all diseases caused by the overexpression of transglutaminase in nerve tissues.

As for cancers, although not part of the present invention, these are found to significantly increase in the level of expression of transglutaminase upon metastasis or to change into forms resistant to chemicals or radiation.

The pharmaceutical composition containing glucosamine for a derivative thereof for use in accordance with the present invention can be applied to mammals that may suffer from diseases due to the activation of transglutaminase, including cattle, horses, sheep, pigs, goats, camels, antelopes, dog, and cats, as well as humans.

The pharmaceutical composition comprising glucosamine or a derivative thereof for use in accordance with the present invention may be used alone or in combination with other pharmaceutical compositions.

The pharmaceutical composition of the present invention may be formulated into various dosage forms. For example, it may be loaded into a capsule without or together with an expedient, a fine solid carrier and/or a liquid carrier. Starch, water, brine, ethanol, glycerol, Ringer's solution, and dextrose solutions may be used as suitable carriers. Reference may be made to the literature (Remington's Pharmaceutical Science, 19thEd., 1995, Mack Publishing Company, Easton PA) upon formulation of the pharmaceutical composition.

Any dosage form, whether oral or non-oral, may be used to administer the formulation of the pharmaceutical composition according to the present invention. Non-oral dosage forms may be injections, coatings, and sprays such as aerosols, with preference for injections and sprays. Also preferable are oral dosage forms.

Examples of the oral dosage forms suitable for the pharmaceutical composition of the present invention include tablets, troches, lozenges, aqueous or emulsive suspensions, powder, granules, emulsions, hard or soft capsules, syrups, and elixirs. For tablets or capsules of the pharmaceutical composition according to the present invention, useful are a binder, such as lactose, saccharose, sorbitol, mannitol, starch, amylopectin, cellulose or gelatin, a carrier, such as dicalcium phosphate, a disintegrant, such as corn starch or sweet potato starch, and a lubricant, such as magnesium.stearate, calcium stearate, sodium stearylfumarate or polyethylene glycol wax, alone or in combination. For capsules, a liquid carrier, such as lipid, may be further used in addition to the above-mentioned compounds.

For non-oral administration, the pharmaceutical composition of the present invention may be formulated into injections for subcutaneous, intravenous, or intramuscular routes, suppositories, or sprays inhalable via the respiratory tract, such as aerosols. Injection preparations may be obtained by dissolving or suspending glucosamine or a derivative thereof, together with a stabilizer or a buffer, in water and packaging the solution or suspension in ampules or vial units. Suppositories are typically made of a suppository base, such as cocoa butter or another glyceride, or a therapeutic laxative in which the active substance, that is, glucosamine or a derivative thereof is diluted. For sprays, such as aerosol, a propellant for spraying a water-dispersed concentrate or wetting powder may be used in combination with an additive.

The pharmaceutical composition of the present invention may be administered via typical routes, such as rectal, local, intravenous, intraperitoneal, intramuscular, intraarterial, transdermal, intranasal, inhalational, intraocular, and subcutaneous routes. For administration via non-oral routes, glucosamine or a derivative thereof in a desired purity is preferably mixed with a pharmaceutically acceptable carrier, that is, a carrier non-toxic at dosage concentrations and amounts and compatible with other ingredients, and formulated into a unit dosage form. In particular, it is required to exclude oxidants and other compounds known to be hazardous to the human body.

Glucosamine or a derivative thereof may be administered along with at least one pharmaceutically acceptable expedient. It will be obvious to those skilled in the art that when the pharmaceutical composition of the present invention is administered to human patients, the total daily dose should be determined through appropriate medical judgment by a physician. The therapeutically effective amount for patients may vary depending on various factors well known in the medical art, including the kind and degree of the response to be achieved, concrete compositions according to whether other agents are used therewith or not, the patient's condition, such as age, body weight, state of health, sex, diet, etc., the frequency, time and route of administration, the secretion rate of the composition, the time period of therapy, etc. For agents suitable for use in the art, reference may be made to the literature (Remington's Pharmaceutical Science, 19thEd., 1995, Mack Publishing Company, Easton PA). Accordingly, the effective dosage of glucosamine or a derivative thereof is preferably determined with reference to the above-mentioned considerations.

A better understanding of the present invention may be obtained through the following examples which are set forth to illustrate, but are not to be construed as the limit of the present invention.

### EXAMPLE 1: in vitro Assay for Inhibition of Transglutaminase Activity

In the presence of various concentrations of transglutaminase, which competes with putrescine, the reaction between [1,4-¹⁴C] putrescine and succinylated casein, catalyzed by transglutaminase, was observed.

A reaction buffer (0.5 ml) was prepared as follows: 0.1 M triacetate pH 7.5, 1 % succinylated casein, 1 mM EDTA, 10 mM CaCl₂, 0.5% lubrol PX, 5 mM DTT, 0.15 M NaCl and 0.5 mCi [1,4-¹⁴C) putrescine (Dupont-New England Nuclear; 118 Ci/mole). To this reaction buffer were added 2 µl of a purified transglutaminase enzyme (Sigma T5398), glucosamine and a 50 mM Tris-acetate buffer so as to yield a final volume of 100 µl, having a glucosamine concentration of 1, 10 or 100 mM. Mixing was followed by reaction at 37°C for 1 hour before termination with 4.5 ml of cold (4°C) 7.5 % TCA. The TCA-precipitates thus formed were filtered through a GF/glass fiber filter, washed with 10 volumes of 5 % TCA and dried, followed by measuring transglutaminase-catalyzed ¹⁴C-putrescine-labelled casein with a β-counter.

The relative inhibition activities (%) of glucosamine against transglutaminase are depicted in FIG. 2. As seen in FIG. 2, the activity of transglutaminase is inhibited in a glucosamine dose-dependent manner.

### EXAMPLE 2: Cell Culture Using BV2 Microglia

LPS-activated murine BV3 microglia cells exhibit the phenotypic and functional characteristics of reactive microglia. The inhibitory effects of glucosamine on transglutaminase were determined by monitoring the activity of the transcriptional factor NF-kB through the measurement of level of NF-kB-inducible iNOS.

The cells were maintained in DMEM (Dulbecco's Modified Eagle's medium) (Invitrogen) supplemented with 10% fetal bovine serum and penicillin/streptomycin in a 5% CO₂, 37°C incubator. BV-2 was activated by treatment with LPS (100ng/ml; Sigma) for 24 hrs.

In order to detect the intracellular activity of transglutaminase, the cells were incubated for 24 hrs in a medium supplemented with 4.5 nmoles of ¹⁴C-putrescine (1.11 X 10⁶ dpm)/ml. Cells were grouped according to treatment without or with 1, 10 or 100 mM of glucosamine. Following LPS treatment for 24 hrs, cells in each group were homogenized with a radioimmunoprecipitation assay buffer (1X PBS, 1% Nonidet P-40, 0.5 % sodium deoxycholate and 0.1 % SDS) containing a protease inhibitor. The homogenates were centrifuged at 100,000 x g for 1 hr. and the supernatants were subjected to iNOS Western blotting. The results are given in FIG. 3A.

In the meanwhile, the pellets were suspended in TENT buffer (50 mM Tris-acetate, 1 mM EDTA, 150 mM NaCl, 1 % Triton-X100) with shaking, and were centrifuged at 10,000 x g for 10 min. The pellets thus obtained were suspended in 0.1 % SDS, 1 mM DTT buffer and boiled for 10 min in a water bath, followed by centrifugation at 100,000 x g for 15 min. The resulting pellets were subjected once again to this procedure. Radioactivity was measured from the pellets using a β-counter, and the results are shown in FIG. 3B.

As apparent from data of FIG. 3A, glucosamine reduces the level of expression of iNOS, which is an LPS-inducible inflammatory indicator.

Under this condition, the *in vivo* activity of transglutaminase is inhibited in a glucosamine dose-dependent manner as clearly shown in FIG. 3B.

A higher degree of crosslinking linkages catalyzed by transglutaminase contains a higher content of ¹⁴C-putrescine therein while a reduced activity of transglutaminase results in a decrease in radioactivity.

After being separated from a 10~20% gradient SDS gel using Tricine buffer (Invitrogen), the Western blotting samples were transferred onto polyvinylidene difluoride membrane (Invitrogen). Western blotting was conducted as usual. An anti-iNOS antibody was purchased from Santa Cruz Co. For a negative control, lactate dehydrogenase was purchased from Research Diagnostics, Inc., Flanders, NJ, U. S. A. The primary and the secondary antibody were used at concentrations of 5 and 0.1 µg/ml, respectively. Blotting was conducted with an enhanced chemiluminescent (Pierce, Milwaukee, WI).

### EXAMPLE 3: Assay in Cell Culture Using SH-SY5Y Neuroblastoma (not part of the invention).

For use in the transfection of transglutaminase, the human neuroblastoma cell SH-SY5Y was purchased from American Type Culture Collection. SH-SY5Y was cultured in DMEM (Dulbecco's modified Eagle's Medium) /Ham F12 medium (50:50) supplemented with 10% heat-inactivated fetal bovine serum, glutamine, and penicillin/streptomycin. In order to avoid clonal mutation, an Flp-InTM system (Invitrogen, Co) was introduced. SH-SY5Y transformed with a mock vector was used as a positive control (Wild) while a full-length transglutaminase gene was cloned in a pcDNA5/FRT vector to prepare SH-SY5Y/TG.

After selection, SH-SY5Y/TG cells were found not to increase in apoptosis as measured by the analysis of the growth of normal cells, the release of free lactate dehydrogenase (LDH), the staining profiles of 4', 6'-diamidino-2-phenylindole dihydrochloride and annexin V, and caspase activity. These results are consistent with those of previous reports teaching that transglutaminase-introduced neuroblastoma cells do not undergo apoptosis to a greater extent than usual as long as they are not exposed to an oxidative condition.

Allowing NF-kB to be usually active therein (J. Biol. Chem. 279, 53725-53735, 2004), the strain thus constructed is widely used as an NF-kB activation model based on the overexpression of transglutaminase. Administration of a transglutaminase inhibitor to this model resulted in the deactivation of NF-kB.

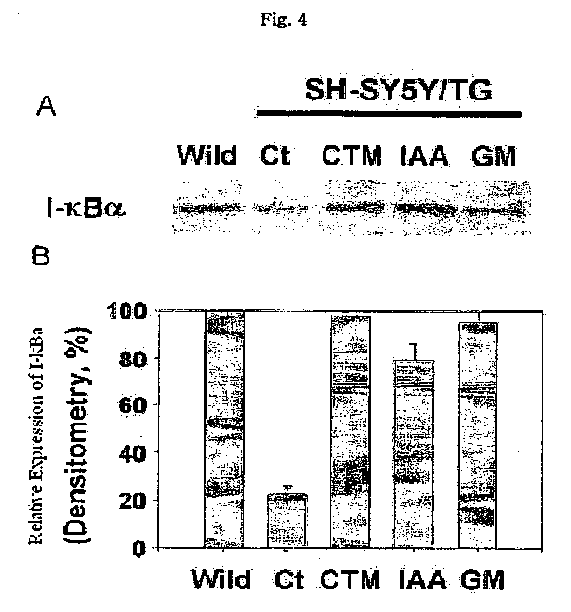

The inhibitory effects of putative transglutaminase inhibitors were investigated by incubating SH-SY5Y/TG cells in the presence of 10 mM glucosamine (GM), 200 µM cystamine (CTM), and 2 nM iodoacetamide (IAA) for 3 hrs, followed by obtaining cytosol fractions with the aid of a nuclear extraction kit (Sigma) from the cells. As a negative control, a cytosol fraction obtained from a cell culture incubated in the absence of any transglutaminase inhibitor was used (Ct). Western blotting was conducted with the cytosol fractions in the same manner as in Example 2, with the exception that I-κBα antibody (Cell Signaling) was used, and the results are given in FIG. 4.

Like other transglutaminase inhibitors, glucosamine was identified as a transglutaminase inhibitor, as is apparent from the data of FIG. 4A, which shows the level of expression of I-κBα through Western blotting analysis.

### Industrial Applicability

As described hitherto, the present invention provides novel uses of glucosamine or a derivative thereof.

Featuring the use of glucosamine or derivatives thereof, the novel uses according to the present invention can be therapeutically effectively and safely applied to patients who suffer from nervous system disorders without causing side-effects.

## Claims

1. Glucosamine or a glucosamine derivative of Chemical Formula 2: 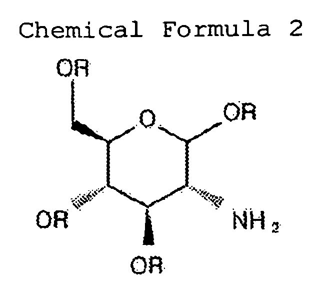 wherein, R is an acyl group having 2 to 18 carbon atoms or a straight or branched alkyl group having 1 to 5 carbon atoms, for use in the treatment of a nervous system disorder.

2. The glucosamine or glucosamine derivative of Chemical Formula 2 for use as defined in claim 1, wherein the nervous system disorder is Alzheimer's disease, Huntington's disease, or Parkinson's disease.

3. A pharmaceutical composition comprising glucosamine or a glucosamine derivative of Chemical 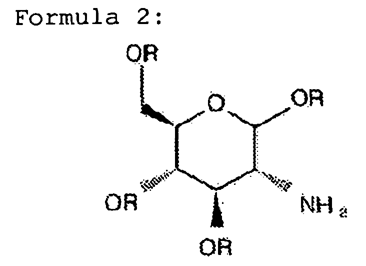 wherein, R is an acyl group having 2 to 18 carbon atoms or a straight or branched alkyl group having 1 to 5 carbon atoms, for use in the treatment of a nervous system disorder.

4. The pharmaceutical composition for use as defined in claim 3, wherein the nervous system disorder is Alzheimer's disease, Huntington's disease, or Parkinson's disease.

5. The pharmaceutical composition for use as defined in claim 3 or 4, further comprising a pharmaceutically acceptable expedient.

## Patentansprüche

1. Glucosamin oder ein Glucosaminderivat mit der chemischen Formel 2: 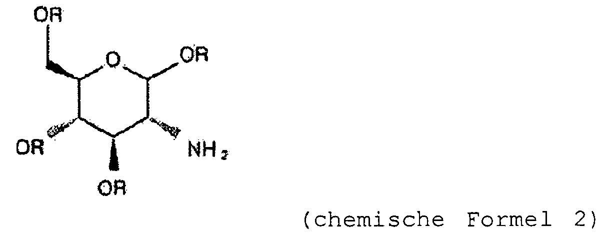 wobei R für eine Acylgruppe mit zwischen 2 und 18 Kohlenstoffatomen oder für eine geradkettige oder verzweigte Alkylgruppe mit zwischen 1 und 5 Kohlenstoffatomen steht, zur Verwendung bei der Behandlung einer Störung des Nervensystems.

2. Glucosamin oder ein Glucosaminderivat mit der chemischen Formel 2 zur Verwendung nach Anspruch 1, wobei es sich bei der Störung des Nervensystems um die Alzheimer-Krankheit, Chorea Huntington oder die Parkinson-Krankheit handelt.

3. Pharmazeutische Zusammensetzung umfassend Glucosamin oder ein Glucosaminderivat mit der chemischen Formel 2: 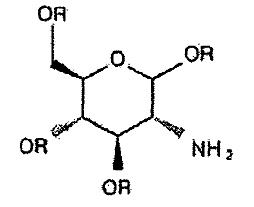 wobei R für eine Acylgruppe mit zwischen 2 und 18 Kohlenstoffatomen oder für eine geradkettige oder verzweigte Alkylgruppe mit zwischen 1 und 5 Kohlenstoffatomen steht, zur Verwendung bei der Behandlung einer Störung des Nervensystems.

4. Pharmazeutische Zusammensetzung zur Verwendung nach Anspruch 3, wobei es sich bei der Störung des Nervensystems um die Alzheimer-Krankheit, Chorea Huntington oder die Parkinson-Krankheit handelt.

5. Pharmazeutische Zusammensetzung zur Verwendung nach Anspruch 3 oder 4, des Weiteren umfassend einen pharmazeutisch verträglichen Hilfsstoff.

## Revendications

1. Glucosamine ou dérivé de glucosamine de formule chimique 2 : 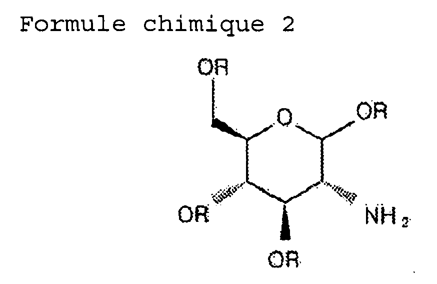 dans laquelle R est un groupe acyle ayant 2 à 18 atomes de carbone ou un groupe alkyle linéaire ou ramifié ayant 1 à 5 atomes de carbone, destiné(e) à être utilisé(e) pour traiter un trouble du système nerveux.

2. Glucosamine ou dérivé de glucosamine de formule chimique 2 destiné(e) à être utilisé(e) selon la revendication 1, dans laquelle/lequel le trouble du système nerveux est la maladie d'Alzheimer, la chorée de Huntington ou la maladie de Parkinson.

3. Composition pharmaceutique comprenant de la glucosamine ou un dérivé de glucosamine de formule chimique 2 : 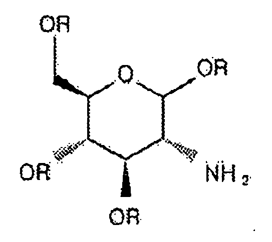 dans laquelle R est un groupe acyle ayant 2 à 18 atomes de carbone ou un groupe alkyle linéaire ou ramifié ayant 1 à 5 atomes de carbone, destinée à être utilisée pour traiter un trouble du système nerveux.

4. Composition pharmaceutique destinée à être utilisée selon la revendication 3, dans laquelle le trouble du système nerveux est la maladie d'Alzheimer, la chorée de Huntington ou la maladie de Parkinson.

5. Composition pharmaceutique destinée à être utilisée selon la revendication 3 ou 4, comprenant en outre un excipient pharmaceutiquement acceptable.
